**Europäisches Patentamt**

**European Patent Office**

**Office européen des brevets**

(19)

(11) Veröffentlichungsnummer: **0 117 438**
**A2**

(12) # EUROPÄISCHE PATENTANMELDUNG

(21) Anmeldenummer: **84100808.9**

(22) Anmeldetag: **26.01.84**

(51) Int. Cl.³: **A 61 L 15/04,** A 61 F 13/00

(30) Priorität: **29.01.83 DE 3302984**

(43) Veröffentlichungstag der Anmeldung: **05.09.84**
**Patentblatt 84/36**

(84) Benannte Vertragsstaaten: **AT CH FR GB IT LI NL SE**

(71) Anmelder: **Sorbexx GmbH Gesellschaft für Adsorptionstechnik und Verbundstoffe, Bayreuther Strasse 3-5, D-8586 Gefrees (DE)**

(72) Erfinder: **Rupprecht, Michael, Dipl.-Ing., Albert-Schweitzer-Strasse 11, D-8586 Gefrees (DE)**

(74) Vertreter: **LOUIS, PÖHLAU, LOHRENTZ & SEGETH, Kesslerplatz 1, D-8500 Nürnberg (DE)**

(54) **Wundabdeckmaterial in Bahnform.**

(57) Es wird ein Wundabdeckmaterial in Bahnform vorgeschlagen, welches aus mindestens zwei ganzflächig verbundenen Lagen besteht.

Eine Lage hat dabei eine offenporige, grobe Oberfläche und besteht aus Schaumstoff, so daß ein Verwachsen mit der Wunde möglich ist. Die andere Lage ist glatt und besteht aus einem nicht in die Wunde einwachsenden Werkstoff.

Außerdem ist in das Wundabdeckmaterial Aktivkohle eingelagert. Es können außerdem in dem Material medizinisch wirksame Stoffe enthalten sein.

## Wundabdeckmaterial in Bahnform

Die Erfindung betrifft ein Wundabdeckmaterial in Bahnform aus mehreren Lagen.

Wundabdeckmaterialien sollen verschiedene Eigenschaften besitzen, um den unterschiedlichen Anforderungen eines solchen Materials entsprechen zu können. Zum einen ist es häufig erwünscht, dass ein gewisses Einwachsen der Abdeckung erfolgt, um auf diese Weise eine mechanische Reinigung der Wunde, beispielsweise beim Abreissen des Verbandes nach einer gewissen Zeit, zu erzielen. Andererseits gibt es jedoch auch Anwendungsfälle, wo ein Einwachsen des Verbandes in die Wunde absolut unerwünscht ist, beispielsweise dann, wenn es darum geht, dass sich erneut eine Haut bildet. Schliesslich besteht bei Wundabdeckungen der Wunsch, einen Angriff von Bakterien oder giftigen Stoffen (Toxinen) auszuschalten.

Bisher ist man in diesem Zusammenhang so vorgegangen, dass für die unterschiedlichen Anwendungsfälle verschiedene Wundabdeckungen mit den entsprechenden Eigenschaften verwendet wurden. Ein Schutz gegen Bakterien bzw. sonstige schädliche Stoffe wurde im allgemeinen durch spezielle, direkt auf die Wunde aufgebrachte Mittel, z.B. Puder, Salben usw., erreicht. Ein derartiges Vorgehen hat mehrere Nachteile. Zum einen wird das Verbinden häufig, insbesondere wenn zusätzliche Mittel aufgebracht werden sollen, sehr umständlich. Zum anderen mussten bisher stets die unterschiedlichsten Abdeckungen vorrätig gehalten werden, um jeweils die richtige Abdeckung einsetzen zu können. Ein weiterer Mangel der bekannten Wundabdeckungen bestand darin, dass sie häufig relativ formsteif waren (sie hatten in vielen Fällen eine nicht dehnbare Gewebe-Trägerschicht), was das Verbinden an bestimmten Körperstellen zusätzlich erschwerte.

Der Erfindung liegt nun die Aufgabe zugrunde, ein Wundabdeckmaterial zur Verfügung zu stellen, welches in beiden vorerwähnten Anwendungsfällen zum Einsatz kommen kann und zudem die Möglichkeit bietet, einen weitgehenden Schutz der Wunde gegen Schadstoffe, Bakterien usw. zu erreichen, gegebenenfalls sogar eine besondere medizinische Wirkung zu erzielen, was für den Anwender den Vorteil einer leichten Handhabung, geringen Lagerhaltung und guten Handhabbarkeit bringt.

Zur Lösung dieser Aufgabe wird erfindungsgemäss ein Wundabdeckmaterial der eingangs erwähnten Art vorgeschlagen, welches sich dadurch auszeichnet, dass es als aus mindestens zwei ganzflächig miteinander verbundenen Lagen bestehendes Verbundmaterial ausgebildet ist, wobei eine die eine Oberfläche bildende erste Lage ein offenporiger, grober Schaumstoff und eine die andere Oberfläche bildende zweite Lage ein glatter, nicht in die Wunde einwachsender Werkstoff ist, und dass in das Verbundmaterial Aktivkohle in Pulver-, Granulat- oder Kugelform eingelagert ist.

Das Wundabdeckmaterial gemäss der Erfindung besitzt also zwei unterschiedliche Oberflächen. Zum einen die Oberfläche des offenporigen, groben Schaumstoffes, die falls erwünscht, in die Wunde einwächst und so eine mechanische Reinigung der Wunde durch Abreissen des Verbandes ermöglicht. Die andere Oberfläche ist dagegen so ausgebildet, dass sie nicht mit der Wunde verwächst. Wird daher das Abdeckmaterial so eingesetzt, dass diese Oberfläche auf der Wunde aufliegt, kann sich ohne Probleme eine einwandfreie, neue Haut bilden. Ein weiteres Merkmal der Abdeckung gemäss der Erfindung ist schliesslich darin zu sehen, dass in das Verbundmaterial Aktivkohle eingelagert ist. Diese Aktivkohle beseitigt zum einen ohne weiteres eventuell auftretene Wundgerüche. Weiterhin hat sich in Versuchen gezeigt, dass durch das Vorhandensein von Aktivkohle der Heilungsprozess beschleunigt wird. Aktivkohle ist leicht und biologisch inaktiv.

Bestimmte Bakterien und Toxine werden durch die Aktivkohle adsorbiert. Dadurch erreicht man deren Beseitigung. Damit unterstützt der Wundverband die Wirkung der Medikamente. Ausserdem können die Bakterien keine Resistenz gegen das verwendete Adsorptionsmedium aufbauen, wie das z.B. bei Antibiotika der Fall ist.

Bei Verwendung der Wundabdeckung nach der Erfindung geht die Epithelbildung schneller und gleichmässiger von statten als bei den bisher bekannten Verbandmaterialien. Schliesslich unterstützt die Einlagerung von Aktivkohle auch die Eindämmung von Infektionen, da die Kohle Toxine entweder durch Adsorption oder Chemiesorption beseitigen bzw. binden kann. Schliesslich lässt sich das Wundabdeckmaterial nach der Erfindung ohne Schwierigkeiten auch derart ausbilden, dass es eine gewisse Dehnbarkeit besitzt, wodurch ein Verbinden an solchen Stellen, bei denen bisher Probleme wegen der starken Wölbung des Körpers usw. auftraten, ohne grosse Schwierigkeiten möglich ist.

Bezüglich der genauen Zusammensetzung des Wundabdeckmateriales hat es sich als zweckmässig gezeigt, wenn die erste Lage aus offenporigem Polyurethan-Schaumstoff besteht. Die zweite Lage kann von einem glatt verdichteten Schaumstoff gebildet sein, wobei dieser wiederum zweckmässig ein Polyurethan-Schaumstoff ist. Die Verwendung zweier Schaumstofflagen für das Wundabdeckmaterial hat insbesondere den Vorzug, dass sich die Lagen leicht und einwandfrei miteinander verbinden lassen und man über die gesamte Dicke des Materials einheitliche Gebrauchseigenschaften erhält.

Es kann jedoch für bestimmte Anwendungsgebiete auch sehr günstig sein, wenn, was ebenfalls im Rahmen der Erfindung liegt, die zweite Lage von einem Stoff aus Kunstfasern, vorzugsweise einem Polypropylen-Vlies gebildet ist. Polypropylen-Fasern haben dabei den Vorzug, dass sie, auch wenn das Vlies direkt auf die offene Wunde gelegt wird, trotzdem auch nach einer bestimmten Tragezeit ein leichtes Ablösen des Wundabdeckmaterials von der Wunde ermöglichen, da das Vlies nicht einwächst.

Es ist nach der Erfindung weiter vorgesehen, dass in dem Verbundmaterial medizinisch wirksame Stoffe enthalten sind, beispielsweise Stoffe, die einer Infektion entgegenwirken.

Die medizinisch wirksamen Stoffe können als Depot-Körperchen in das Verbundmaterial eingelagert sein, in welchem Falle sich eine kontinuierliche Abgabe der wirksamen Stoffe über einen längeren Zeitraum unmittelbar und direkt in die Wunde erreichen liesse.

0117438

Grundsätzlich kann die Aktivkohle und können gegebenenfalls die medizinisch wirksamen Stoffe in beliebiger Weise in dem Wundabdeckmaterial festgelegt sein, z.B. mittels entsprechender Klebstoffe usw.. Eine besonders sichere Verankerung erreicht man allerdings dann, wenn die Aktivkohle und gegebenenfalls die medizinisch wirksamen Stoffe zwischen zwei benachbarten Lagen des Verbundmaterials festgelegt sind. In einem derartigen Falle ist insbesondere auch die Gewähr dafür gegeben, dass nicht eventuell Aktivkohle-Partikel oder die medizinisch wirksamen Stoffe selbst direkt in die Wunde gelangen, was unter Umständen unerwünschte Nebenwirkungen zur Folge haben könnte.

Schliesslich liegt es im Rahmen der Erfindung, dass die Aktivkohle-Partikel in die erste Lage, d.h. die offenporige Schaumstoff-Lage, eingelagert sind, weil sie dann ihre Wirkung auf die offene Wunde besser entfalten können.

Es sei schliesslich noch darauf hingewiesen, dass sich bei einem Wundabdeckmaterial gemäss der Erfindung eine genau definierte Wasserdampf- und Luftdurchlässigkeit erreichen lässt, wodurch eine zu hohe Feuchtigkeitsabgabe und damit eine unerwünscht rasche Austrocknung der Wunde verhindert werden kann. Da es sich bei dem Wundabdeckmaterial um ein solches in Bahnform handelt und da die verschiedenen Lagen ganzflächig miteinander verbunden sind, lassen sich aus dem Wundabdeckmaterial Abdeckungen in beliebigen Formen und Grössen zuschneiden, wodurch ein universeller Einsatz des Materiales nach der Erfindung möglich ist. Ausserdem ist auch die Möglichkeit gegeben, das Wundabdeckmaterial ohne weiteres zu sterilisieren, beispielsweise durch Dampf-, Gas- oder Strahlungs-Sterilisation, und es dann steril zu verpacken, so dass beim Verbinden auch keine besonderen Desinfektionsmittel - soweit der Verband betroffen ist - verwendet werden müssen.

Nachstehend werden zwei Beispiele für Wundabdeckmaterialien gemäss der Erfindung erläutert.

Beispiel 1
----------

Es wird ein Wundabdeckmaterial verwendet, bei dem die erste Lage aus einem offenporigen Polyurethan-Weichschaum besteht. Diese Lage ist ganzflächig mit einer glatten Polypropylen-Vlies-Lage verbunden. Die Dicke der gesamten Wundabdeckung liegt bei etwa 1,5 bis 3 mm, wobei die Dicke der Schaumstoff-Lage erheblich höher ist als die der Vlies-Lage.

In die Polyurethan-Schaum-Komponente ist eine hochaktivierte Kohle in Pulver-, Granulat- oder Kugelform fest eingelagert, z.B. mittels eines besonderen Haftmittels oder durch entsprechende Aktivierung (Anlösung) der Zellwände des Polyurethan-Schaumstoffes.

Bei Verwendung einer derartigen Wundabdeckung wird, wenn eine mechanische Reinigung der Wunde gewünscht ist, die Lage aus offenporigem Polyurethan-Weichschaum direkt auf die Wunde aufgelegt. Wird dagegen das Polypropylen-Vlies, welches sehr hautsympatisch ist, direkt auf die Wunde gelegt, so ist nach einer bestimmten Tragezeit ein leichtes Ablösen möglich.

Die Wundgerüche werden bei Verwendung des Verbandes durch die Aktivkohle beseitigt. Bei Auflegen der Vlies-Schicht auf die Wunde erfolgt eine besonders rasche Epithelbildung, wie von der Anmelderin in Rechts/Links-Vergleichen mit konventionellen Wundabdeckungen festgestellt wurde. Schliesslich erreicht man eine Infektionseindämmung, da die Kohle die bestimmten Wundbakterien, Toxine und Pyrogene beseitigt bzw. bindet.

Beispiel 2
----------

Bei einer anderen Ausführungsform des Wundabdeckmaterials besteht die erste Lage wiederum aus einem groben, offenporigen Polyurethan-Weichschaum. Die zweite Lage ist ebenfalls aus Polyurethan-Weichschaum hergestellt, wobei dieser Schaum jedoch zumindest an seiner Oberfläche glatt verdichtet ist. Die Stärke der groben, offenporigen Lage ist üblicherweise grösser als die der glatt verdichteten Lage. Die erste Lage kann beispielsweise etwa 1 mm, die zweite Lage ca. 0,5 mm dick sein, so dass sich eine Gesamtstärke für das Material von ca. 1,5 mm ergibt.

In das Verbundmaterial ist wiederum hochaktive Kohle in Pulver-, Granulat- oder Kugelform eingelagert, wobei die Möglichkeit gegeben ist, die Aktivkohle zwischen die beiden Polyurethan-Schaumstoff-Lagen einzubringen.

Bei Verwendung eines derartigen Materials wird die offenporige Seite, wenn eine mechanische Reinigung gewünscht ist, wiederum direkt auf die Wunde gelegt, wobei dann von dieser Lage die entstehenden bzw. bereits vorhandenen Bakterien aufgenommen und von der Wunde ferngehalten werden könnten. Durch die Verdichtung der zweiten Lage, d.h. das Schliessen der in dieser Lage vorhandenen Poren, wird erreicht, dass von aussen nach innen, d.h. zur Wunde zu, keine Bakterien eindringen können. Es ist somit bei Verwendung des Abdeckmaterials derart, dass die offenporige Lage auf der Wunde liegt, eine mechanische Reinigung der Wunde möglich, indem man nämlich den Verband in die Wunde einwachsen lässt und ihn dann entfernt.

Üblicherweise wird man den Verband nach ca. 7 bis 12 Tagen entfernen und dann einen neuen Verband aus dem gleichen Wundabdeckmaterial anlegen, wobei dann aber die offenporige Lage nach aussen, die glatte Lage aus verdichtetem Schaum zur Wunde hin kommt. Auf diese Weise wird die Hautbildung begünstigt. Ausgeschiedene Toxine und Bakterien und Pyrogene werden von der Aktivkohle ständig beseitigt.

Insbesondere bei Verwendung eines Wundabdeckmaterials gemäss dem Beispiel 2 wäre an den Einbau eines Medikamenten-Depots, vorzusgweise in Form von kleinen Körnchen (Mikrokapseln), zu denken, das in der auch die Aktivkohle enthaltenden Schicht sitzen könnte. Bei Verwendung derartiger Medikamenten-Depots liesse sich eine kontinuierliche Diffusion der Medikamente unmittelbar und direkt in die Wunde erreichen, wobei durch Änderung der Konzentration der medizinisch wirksamen Stoffe selbstverständlich auch die gesamte Wirksamkeit dieser Stoffe variiert werden kann.

Es sei zu den beiden Beispielen noch darauf hingewiesen, dass die verwendeten Rohstoffe, nämlich bei den Beispielen Polyurethan-Weichschaum, Polypropylen-Vlies, Aktivkohle und Haftmittel toxikologisch unbedenklich sind.

- 1 -

Patentansprüche:

1. Wundabdeckmaterial in Bahnform aus mindestens zwei ganzflächig miteinander verbundenen Lagen, von denen eine erste saugfähig ist und die zweite, die eine Oberfläche bildende Lage aus einem glatten, nicht in die Wunde einwachsenden Werkstoff besteht, dadurch gekennzeichnet, dass die erste Lage die andere Oberfläche bildet und ein offenporiger, grober Schaumstoff ist, und dass in das Verbundmaterial aus den Lagen Aktivkohle in Pulver-, Granulat- oder Kugelform eingelagert ist.

2. Wundabdeckmaterial nach Anspruch 1, dadurch gekennzeichnet, dass die erste Lage aus offenporigem Polyurethan-Schaumstoff besteht.

3. Wundabdeckmaterial nach Anspruch 1 oder 2, dadurch gekennzeichnet, dass die zweite Lage von einem glatt verdichteten Schaumstoff gebildet ist.

0117438

4. Wundabdeckmaterial nach Anspruch 3,
   dadurch gekennzeichnet,
   dass der Schaumstoff ein Polyurethan-Schaumstoff ist.

5. Wundabdeckmaterial nach Anspruch 1 oder 2,
   dadurch gekennzeichnet,
   dass die zweite Lage von einem Stoff aus Kunstfasern,
   vorzugsweise einem Polypropylen-Vlies, gebildet ist.

6. Wundabdeckmaterial nach einem der vorhergehenden Ansprüche, dadurch gekennzeichnet,
   dass in dem Verbundmaterial medizinisch wirksame Stoffe enthalten sind.

7. Wundabdeckmaterial nach Anspruch 6,
   dadurch gekennzeichnet,
   dass die medizinisch wirksamen Stoffe als Depot-Körperchen in das Verbundmaterial eingelagert sind.

8. Wundabdeckmaterial nach einem der vorhergehenden Ansprüche, dadurch gekennzeichnet,
   dass die Aktivkohle und gegebenenfalls die medizinisch
   wirksamen Stoffe zwischen zwei benachbarten Lagen des
   Verbundmaterials festgelegt sind.

9. Verbundmaterial nach einem der Ansprüche 1 bis 8,
   dadurch gekennzeichnet,
   dass die Aktivkohle-Partikel in die erste Lage eingelagert sind.